(19) Europäisches Patentamt
European Patent Office
Office européen des brevets

(11) **EP 0 803 760 B1**

(12) **EUROPÄISCHE PATENTSCHRIFT**

(45) Veröffentlichungstag und Bekanntmachung des Hinweises auf die Patenterteilung:
**15.09.2004  Patentblatt 2004/38**

(51) Int Cl.[7]: **G02F 1/141**, G02F 1/135, A61F 9/06, H04N 5/238

(21) Anmeldenummer: **97101584.7**

(22) Anmeldetag: **01.02.1997**

(54) **Vorrichtung zur lokalen Abschwächung der Lichtintensität**

Device for the local attenuation of the light intensity

Dispositif pour l'atténuation locale de l'intensité de la lumière

(84) Benannte Vertragsstaaten:
**AT BE CH DE DK ES FI FR GB GR IE IT LI LU MC NL PT SE**

(30) Priorität: **24.04.1996  DE 19616323**

(43) Veröffentlichungstag der Anmeldung:
**29.10.1997  Patentblatt 1997/44**

(73) Patentinhaber: **Deutsche Telekom AG
53113 Bonn (DE)**

(72) Erfinder:
- **Dultz, Wolfgang, Prof. Dr.
  65936 Frankfurt/M. (DE)**
- **Onokhov, Arkadii, Dr.
  198097 St. Petersburg (RU)**
- **Beresnev, Leonid, Dr.
  117393 Moskau (RU)**
- **Haase, Wolfgang, Prof. Dr.
  64354 Reinheim (DE)**

(56) Entgegenhaltungen:
**EP-A- 0 326 467      US-A- 5 073 010
US-A- 5 121 238**

- **B.LANDRETH ET AL.: "Operating characteristics of optically addressed spatial ligth modulators incorporating distorted helix ferroelectric liquid crystals" JAPANESE JOURNAL OF APPLIED PHYSICS, Bd. 30, Nr. 7, Juli 1991, Seiten 1400-1404, XP002074766**
- **GUENA M ET AL: "120 X 100 pixel antiblooming array based on optically addressed ferroelectric liquid-crystal cells" OPTICS LETTERS, Bd. 19, Nr. 13, 1. Juli 1994, Seiten 1001-1003, XP000454706**
- **BERESNEV L.A. ET AL SOV. TECH. PHYS. LETT. Bd. 14, 1988, Seiten 117 - 118**
- **TOMILIN M.G. ET AL MOL. CRYST. LIQ. CRYST. Bd. 222, 1992, Seiten 119 - 124**

**Beschreibung**

Die Erfindung betrifft eine Vorrichtung zur lokalen Abschwächung der Lichtintensität im Sehfeld des menschlichen Auges, von Video- oder Photokameras oder dergleichen nach dem Oberbegriff des Patentanspruchs 1 2 oder 3.

In der modernen Telekommunikation sind optische Methoden zur Übertragung und Bearbeitung von Informationen attraktiv und aussichtsreich, da sie außergewöhnliche Vorteile gegenüber allen anderen Methoden bieten. Zur Registrierung der optischen Informationen aus Lichtquellen unterschiedlicher Intensitäten (Laser, Sonne, Lampen u.s.w.) ist die Beobachtung und Aufzeichnung von optischen Bildern durch das Auge, Videokameras und sonstige lichtempfindliche Vorrichtungen erforderlich.

Die photoempfindlichen Matrizen müssen vor Blendung bis hin zur Beschädigung durch Lichteinfall zu hoher Intensität geschützt werden. Die üblicherweise diesbezüglich verwendeten Sicherheitsvorrichtungen haben zahlreiche Nachteile: neutrale oder polarisierende Filter verringern proportional zur Helligkeit von störendem Licht auch die Helligkeit der zu beobachtenden Objekte. Wird die Belichtung verringert, werden diese Filter mechanisch entfernt. Die bekannten Brillen mit automatischer Umschaltung auf absorbierende Filter bei starkem Lichteinfall schränken das gesamte Sichtfeld ein. Eine bessere Schutzwirkung ist die lokale Abschwächung der Helligkeit stark belichteter Objekte, die sich im Sehfeld des Auges, der Videokamera u.s.w., befinden, ohne dabei gleichzeitig schwach belichtete Objekte zu unterdrücken.

Die Lösung wird mit Hilfe von optisch adressierbaren räumlichen Lichtmodulatoren OASLM erreicht. Sicherheitsbrillen, die optisch adressierbare räumliche Lichtmodulatoren OASLM verwenden, werden von M.G. Tomilin, A.P. Onokhov und D. Yu. Polushkin in Mol Cryst. Lq. Crys., 222, 119, (1992), beschrieben. In diesem Fall wurde der Verdrillungseffekt in nematischen Flüssigkristallen als das auf das Licht wirkende Medium benutzt. Der grundsätzliche Nachteil der Verwendung von nematischen Flüssigkristallen besteht in der relativ langsamen Schaltzeit (ca. $10^{-2}$ s). Dies führt zum Verwackeln (Verwischen) des unterdrückten Bildes während der Bildbewegung von hellen Objekten in optisch adressierbaren räumlichen Lichtmodulatoren OASLM bzw. zur Blendung des Auges oder der Videokamera.

Dieser Fachaufsatz offenbart also eine Augenschutzbrille, etwa für Schweißarbeiten, die einen zwischen einem Objektiv und einem Okular angeordneten optisch adressierbaren räumlichen Lichtmodulator aufweist. Der Lichtmodulator umfasst zwei Polarisatoren, zwischen denen sich eine auf der Objektivseite mit einer transparenten photoleitenden Schicht versehene Flüssigkristallzelle befindet, welche von zwei transparenten Platten begrenzt wird, auf denen Elektroden angebracht sind. Die Flüssigkristallzelle weist dabei zwischen den Platten einen chiralnematischen Flüssigkristall auf.

Ein weiterer Fachaufsatz von Beresnev L.A. et al., veröffentlicht in Sov. Tech. Phys. Lett. 14, S. 117-118 (1988), offenbart einen optisch adressierbaren Lichtmodulator mit einer auf einem ferroelektrischen Flüssigkristall basierenden Flüssigkristallzelle. Es handelt sich bei dem hierbei verwendeten ferroelektrischen Flüssigkristall um einen Flüssigkristall in der smektischen C*-Phase mit deformierbarer ferroelektrischer Helix (DHF-LC), dessen Helixachse parallel zu den zellbegrenzenden Platten angeordnet ist.

Der Erfindung liegt die Aufgabe zugrunde, eine verbesserte und schnellere Vorrichtung zur lokalen Abschwächung der Lichtintensität für die verschiedensten Anwendungen zu schaffen, die dazu die Deformation der ferroelektrischen Helix nutzt.

Die erfindungsgemäße Lösung der Aufgabe ist insbesondere im Kennzeichen des Patentanspruchs 1, 2 oder 3 charakterisiert.

Weitere Merkmale und Ausgestaltungen sind in den Kennzeichen der Patentansprüche 4 bis 10 angegeben.

Bei der in dieser Erfindung vorgeschlagenen Vorrichtung wird ein viel schnellerer elektrooptischer Effekt in ferroelektrischen Flüssigkristallen ausgenutzt, nämlich die Deformation der ferroelektrischen Helix (DHF Effekt), der von L.A. Beresnev et al in dem oben genannten Fachaufsatz in Liquid Crystals, 5, S. 1171, (1989) beschrieben wird. Dieser Effekt ist für chirale smektisch-C-Materialien mit einer sehr kurzen Steighöhe $p_o$ der Helix (0.3μm oder weniger) charakteristisch und besitzt große Vorteile gegenüber den elektrooptischen Effekten in Nematen.

Die Sicherheitsvorrichtung nach der vorliegenden Erfindung ist bedeutend schneller als Vorrichtungen, basierend auf nematischen Flüssigkeitskristallen. Die Betriebsfrequenz liegt im Bereich von ca. $10^2$ bis $10^3$ Hz anstelle von 1-20 Hz bei den Nematen. Dadurch ergibt sich die Möglichkeit, das Verwackeln (Verwischen) des Bildes während der Beobachtung von sich stark bewegenden belichteten Objekten sowie die Blendung des Auges oder der Videokamera zu vermeiden.

Die Erfindung wird im folgenden anhand von in der Zeichnung dargestellten Ausführungsbeispielen näher beschrieben.

In der Zeichnung bedeuten:

Fig. 1    allgemeine Darstellung einer Brille mit lokaler Abschwächung hoher Lichtintensität;

Fig. 2a-c    Verwendung von DHF-LC-Material mit einem molekularen Neigungswinkel $\theta_o$ = 45°;

Fig. 3a+b    Verwendung von DHF-LC-Material mit einem molekularen Neigungswinkel $\theta_o$ <

45°;

Fig. 4 optisches Schema einer Brille mit schnellem Verschluß;

Fig. 5 optisches Schema einer Brille mit schnell umschaltbarem "Kompensator" $FLC_2$;

Fig. 6a-c Verwendung eines Kompensators $FLC_2$ mit einem molekularen Neigungswinkel von $\theta_c = 45° - 0_o$;

Fig. 7 Anwendung eines $FLC_2$-Kompensators mit einem molekularen Neigungswinkel $\theta_c = \theta_o$;

Fig. 8a-d ausgewähltes Bild des Testzielbildes 0683 und

Fig. 9 Arbeitsweise einer Sicherheitsvorrichtung mit lokaler Abschwächung der Helligkeit von Bildern stark belichteter Objekte.

Im folgenden und in der Zeichnung werden folgende Bezugszeichen und zugehörige Begriffe verwendet:

1 Auge und/oder Videokamera
2 Verschluß
3 Kompensator
$L_{1-3}$ Linsen
$P_{1-3}$ Polarisatoren
PC Photoleiter
FLC ferroelektrische Flüssigkristalle
OASLM optisch adressierbare räumliche Lichtmodulatoren
E Spannung

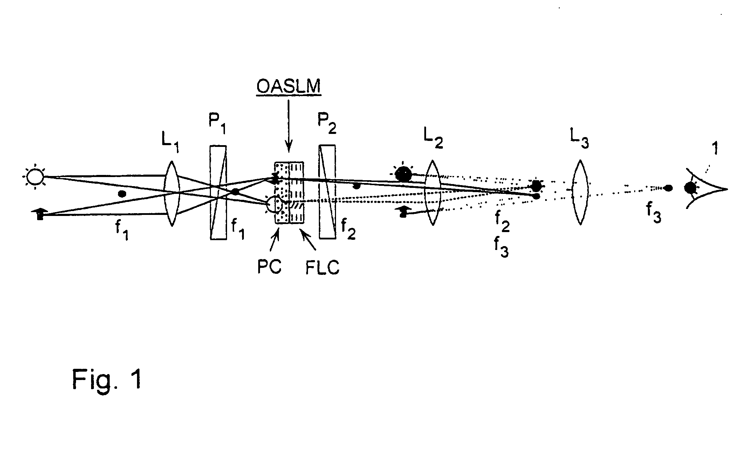

Fig. 1 zeigt eine allgemeine Darstellung einer Brille mit lokaler Abschwächung hoher Lichtintensität mit Hilfe von optisch adressierbaren räumlichen Lichtmodulatoren OASLM. Sie besteht außerdem aus Linsen $L_1$, $L_2$, $L_3$, Polarisatoren $P_1$, $P_2$, Photoleitern PC und ferroelektrischen Flüssigkristallen FLC. Als Ausgang wird folgendes Scenario vorausgesetzt: stark belichtetes Bild - die Sonne, schwach belichtetes Bild - ein Haus. Nach Durchgang durch den Polarisator $P_2$ ist die Intensität des Abbildes der Sonne, verglichen mit der des Hauses, reduziert.

Es folgt die detaillierte Beschreibung des Prinzips und der Funktionsweise der Vorrichtung sowie der einzelnen Bauteile anhand der Fig. 1.

Der in Fig. 1 gezeigte optisch adressierbare räumliche Lichtmodulator OASLM beruht auf einer photoleitenden Schicht PC und einem Flüssigkristallfilm FLC. Bei einer verdrillten nematischen Schicht TN, die die photoleitende Schicht PC berührt, kann das Licht von schwach belichteten Objekten ("Haus") durch die

gekreuzten Polarisatoren $P_1$ und $P_2$ treten. Das Licht von stark belichteten Objekten ("Sonne"), fokussiert auf die Schicht PC, induziert den lokalen Übergang vom verdrillten zum homeotropen Zustand in der Schicht FLC aufgrund der extern an den optisch adressierbaren räumlichen Lichtmodulator OASLM angelegten Spannung. Der Übergang verdrillt - homeotrop - vollzieht sich nur an der Stelle des optisch adressierbaren räumlichen Lichtmodulators OASLM, an der die Leitfähigkeit des Photoleiters PC aufgrund der starken Belichtung zunimmt ("Sonne"). An dieser Stelle tritt das Licht nicht durch den Polarisator $P_2$, die Helligkeit des stark belichteten Objekts ("Sonne") ist im sich ergebenden Bild abgeschwächt. Die Linsen $L_2$ und $L_3$ stellen das einfachste "Auge" 1 zur Betrachtung dieses Bildes dar. Die optische Auslegung des Systems kann eventuell komplizierter sein, abhängig von den Anforderungen an Größe und Form der Brillen (Prismen, Spiegel u.s.w. können eingebaut werden).

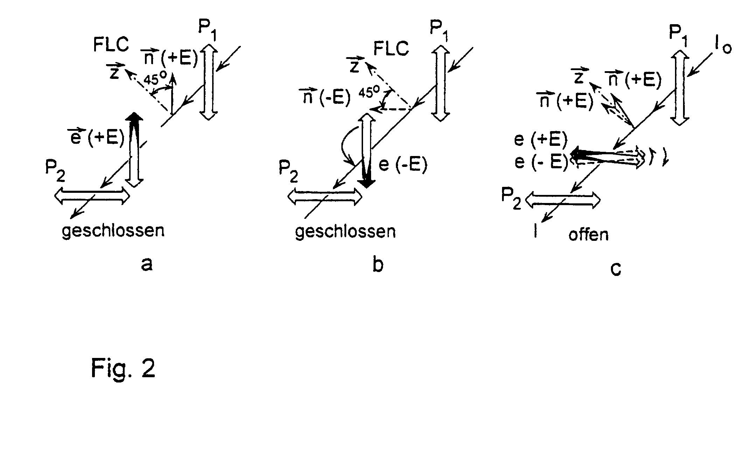

Unter Anwendung des DHF-Effektes treten im optisch adressierbaren räumlichen Lichtmodulator OASLM drei extreme Zustände der mittleren optischen Indicatrix <n> auf (siehe auch Fig. 2a-c), was von B.I. Ostrovski, A.Z. Rabinovich und V.G. Chigrinov in "Advances in Liquid Crystal Research and Applications", herausgegeben von L. Bata, Pergamon Press, Oxford - Akad. Kiado, Budapest - 1980, S. 469, beschrieben wurde: (1) bei positiver Spannung +E (offen) ist die Abweichung von n gleich $+\theta_o$ bezüglich der Richtung z, (2) bei negativer Spannung -E (offen) wird die optische Achse n um den Wert $-\theta_o$ abgelenkt, hier ist $\theta_o$ der molekulare Neigungswinkel in der smektischen Phase C* und n ist die Richtung der Moleküllängsachse (Direktor); (3) bei niedrigen Spannungen wird die Achse < n > der mittleren optischen Berechnungsindicatrix gering und proportional zur angelegten Spannung aus der Richtung z abgelenkt.

Zwei extreme Positionen $+\theta_o$ und $-\theta_o$ werden für die stark belichteten Bereiche des optisch adressierbaren räumlichen Lichtmodulators OASLM realisiert. Daher überschreitet die extern angelegte Spannung, wirkend auf die Schicht FLC, den Schwellenwert für das Aufwinden der Helix.

Fig. 2 zeigt die Wirkung bei Verwendung von DHF-LC-Material mit einem molekularen Neigungswinkel $\theta_o = 45$. a,b - Ebene des polarisierten Lichtes e ($\pm E$) für positive (a) bzw. negative (b) Polarität der Betriebsspannung. Für stark belichtete Bereiche im optisch adressierbaren räumlichen Lichtmodulator OASLM ist der Lichtdurchgang gesperrt. c - kleine Abweichung der Ebenen von e ($\pm E$) von der ursprünglichen Richtung z der Helixachsen (=Reibungsrichtung) für Licht, das von den schwach belichteten Bereichen im optisch adressierbaren räumlichen Lichtmodulator OASLM ausgeht.

Bei einer Anordnung der Schicht FLC für einen molekularen Neigungswinkel $\theta_o = 45°$ nach Fig. 2a und b wird hohe Lichtintensität für beide Polaritäten der Antriebsspannung dann unterdrückt, wenn die Reibrichtung z mit dem Polarisator $P_1$ einen Winkel von 45° bildet. In den schwach belichteten Bereichen des optisch adressierbaren räumlichen Lichtmodulators OASLM verläuft die mittlere optische Indicatrix nahezu parallel zur Richtung der ungestörten Helix z (Fig. 2c), was zur Folge hat, daß fast das gesamte Licht übertragen wird. Die Polarisationsebene des Lichtes $\vec{e}$ (±E) wird dabei um 90° gedreht.

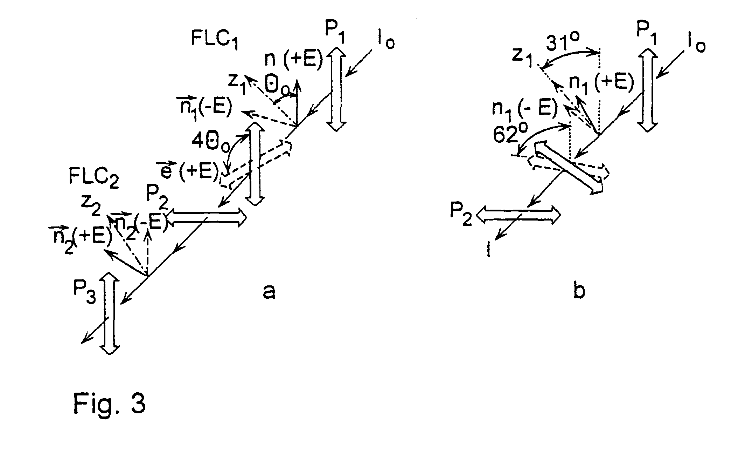

Fig. 3 zeigt die Wirkungsweise bei Verwendung von DHF-LC-Material mit molekularem Neigungswinkel $\theta_o < 45°$. a - Ebenen des polarisierten Lichtes $\vec{e}$ (±E) für positive bzw. negative Polarität der Antriebsspannung in stark (a) und schwach (b) belichteten Bereichen im optisch adressierbaren räumlichen Lichtmodulator OASLM. Für positive Polarität (±E) verläuft der Direktor $n_1$ in der Schicht $FLC_1$. Für negative Polarität (-E) verläuft der Direktor $n_2$(-E) des Verschlusses 2 in Koinzidenz mit dem Polarisator $P_2$. Dadurch fällt durch den Polarisator $P_3$ kein Licht.

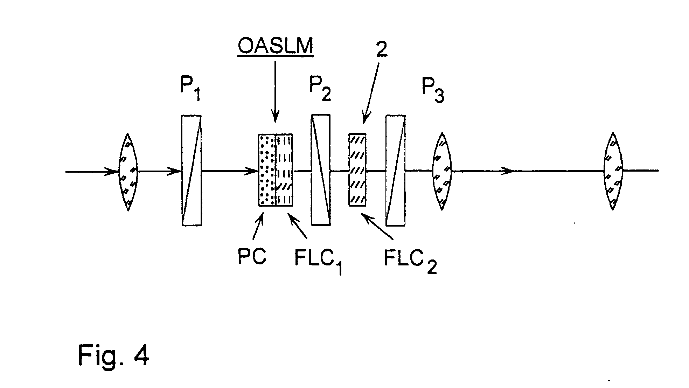

In den Fig. 3 und 4 wird die Situation für $\theta_o \neq 45°$ dargestellt. Bei positiver Polarität +E der Betriebsspannung wird das übertragene Licht in den stark belichteten Bereichen vom optisch adressierbaren räumlichen Lichtmodulator OASLM vollkommen unterdrückt, Fig. 3a. Bei negativer Polarität der Betriebsspannung wird der Schnellverschluß 2 nach Fig. 4 benutzt, um Auge und/oder Videokamera 1 nicht zu blenden.

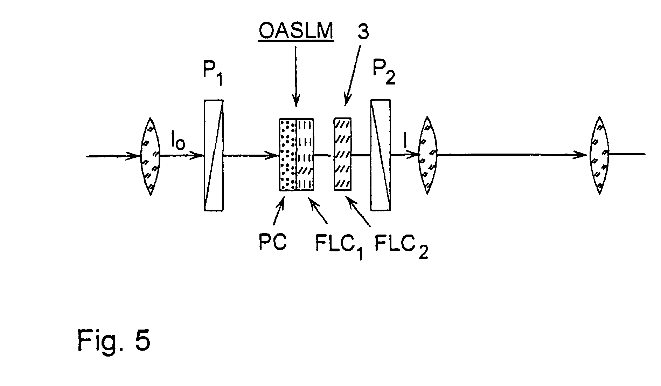

In Fig. 5 ist das optische Schema der Brille bzw. Vorrichtung mit dem schnellen umschaltbaren "Kompensator" 3 dargestellt, wo die Schicht $FLC_1$ einen molekularen Neigungswinkel von $\theta_o < 45°$ einnimmt. Die zweite Schicht $FLC_2$ funktioniert als umschaltbarer "Kompensator" 3, der die Polarisationsebene $\vec{e}$ (-E) des Lichtes, das von den stark belichteten Flächen des Photoleiters in der Schicht $FLC_1$ ausgeht, in Richtung $U_1$ (+E) zurückdreht. In diesem Fall werden bei beiden Polaritäten der Mäanderspannung die stark belichteten Bilder völlig unterdrückt.

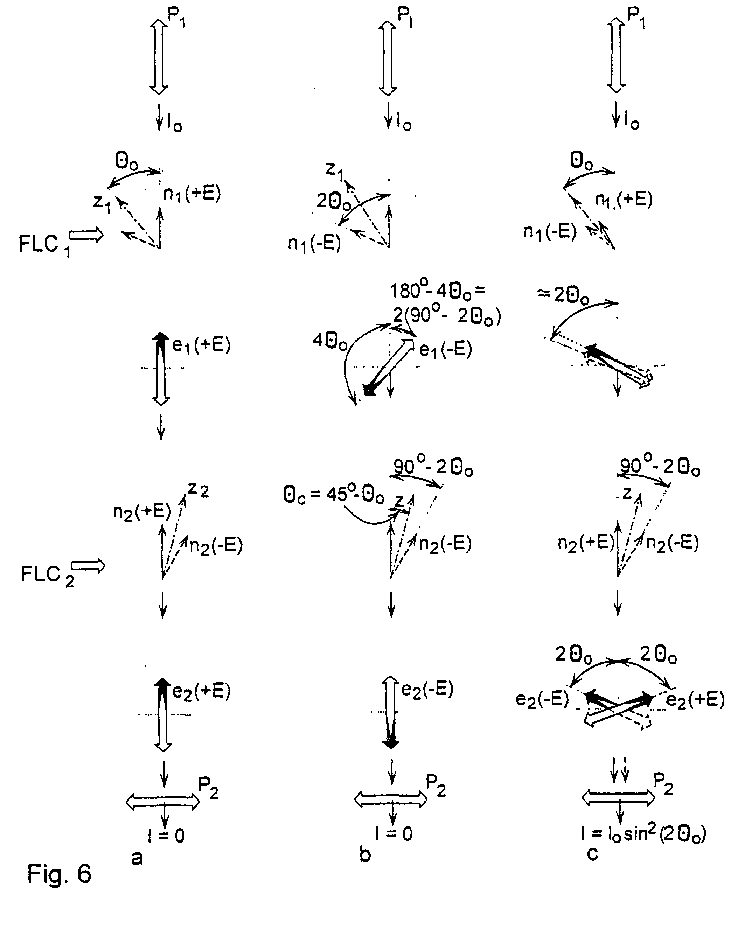

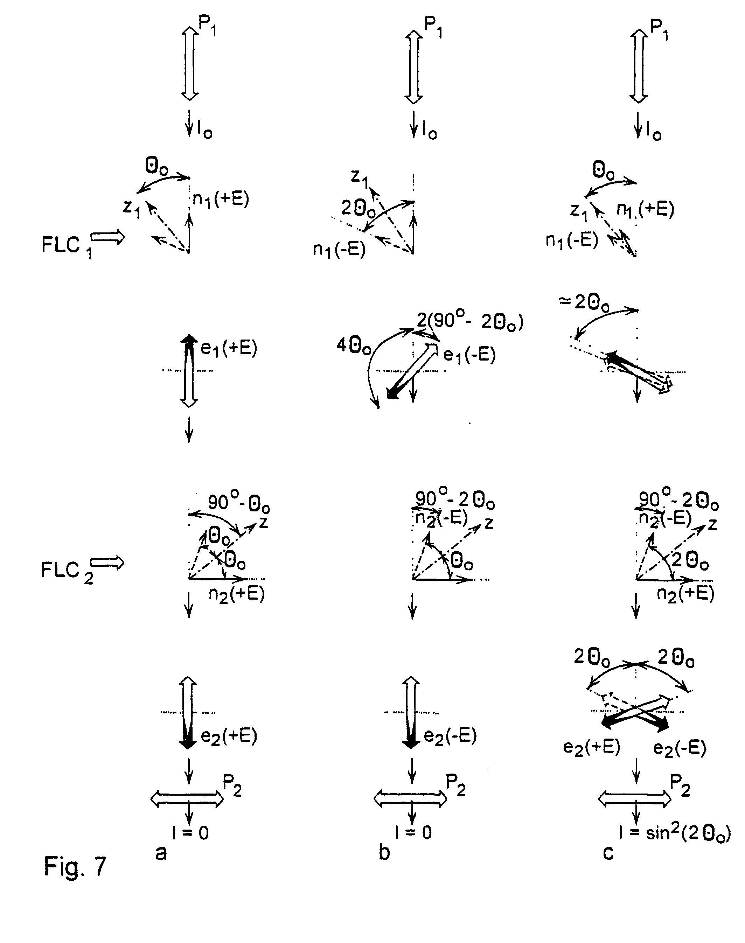

In den Fig. 6a-c und 7a-c wird das Schema der optischen Umformungen in der Vorrichtung gezeigt, wobei als Kompensator der ferroelektrische smektische Flüssigkristall einer C-Phase mit einem molekularen Neigungswinkel $\theta_c = 45° - \theta_o$ bzw. $\theta_c = \theta_o$ benutzt wird.

Die Fig. 6a und b zeigen die Lage des Direktors $n_1$ (±E) der ersten Schicht $FLC_1$. Die Ebene des polarisierten Lichtes $e_1$ (±E), das durch die Schicht $FLC_1$ infolge positiver bzw. negativer Polarität der angelegten Spannung E in stark belichteten Zonen des optisch adressierbaren räumlichen Lichtmodulators OASLM tritt, ist gezeigt und $n_2$ (±E) - die entsprechende Lage des Direktors $n_2$ der Schicht $FLC_2$ (entspricht Kompensator 3). Bei beiden Polaritäten steht die Ebene des polarisierten Lichtes $e_2$ (±E), das durch die Schicht $FLC_2$ geht, senkrecht zu der des Polarisators $P_2$. Deshalb ist in stark belichteten Bereichen des optisch adressierbaren räumlichen Lichtmodulators OASLM der Lichtdurchtritt verhindert.

In Fig. 6c ist die kleine Abweichung des Direktors $n_1$ (±E) von der Richtung $z_1$ der Helixachse in der Schicht $FLC_2$ in schwach belichteten Bereichen des optisch adressierbaren räumlichen Lichtmodulators OASLM zu sehen. Die Lichtintensität I, die von diesen Bereichen ausgeht, ist für beide Spannungspolaritäten $I - I_o \sin^2 (2\,\theta_o)$ gleich. $I_o$ - Intensität des Lichtes, das auf den optisch adressierbaren räumlichen Lichtmodulator OASLM fällt.

Die Fig. 7 zeigt eine Anwendung des $FLC_2$-Kompensators 3 mit einem molekularen Neigungswinkel $\theta_c = \theta_o$. Die sich ergebende Intensität des Lichtes I, die durch den Polarisator $P_2$ von schwach belichteten Bereichen des optisch adressierbaren räumlichen Lichtmodulators OASLM ausgeht, ist gleich $I = I_o \sin_2(2\,\theta_o)$. Verglichen mit dem Fall $\theta_c = 45° - \theta_o$ findet nur eine Phasenänderung der Lichtwelle um 180° statt.

In den in den Fig. 6a-c und 7a-c gezeigten Fällen erfüllt die Dicke der beiden Schichten FLC die Bedingung $\Delta nd = \lambda/2 + N\lambda$, wobei $\lambda$ die Lichtwellenlänge ist. Beim "Kompensator" $FLC_2$ kann das elektroklinische Material, zum Beispiel die chirale smektische A-Phase mit einem relativ kleinen induzierten Neigungswinkel $\theta_c = 5° + 10°$, verwendet werden. Der erforderliche Wert des Winkels $\theta_c = 45° - \theta o$ kann durch Änderung der Spannungsamplitude, angewendet auf die Schicht $FLC_2$, eingepasst werden.

Als illustratives Beispiel der Vorrichtung zur lokalen Abschwächung der Intensität des Lichts nach der vorliegenden Erfindung wird der Betrieb der Sicherheitsvorrichtung auf der Grundlage des optischen Schemas mit "Verschluß" 2, gezeigt in den Fig. 3 und 4, vorgestellt. Optisch adressierbare räumliche Lichtmodulatoren OASLM, die in diesem Schema verwendet werden, bestehen aus zwei Quarzplatten mit transparenten Indium-Zinn-Oxidelektroden. Bei einer der Elektroden wird ein quasi-amorpher ZnSe-Film in einer Dicke von 1μm als photoleitende Schicht verwendet, in Fig. 4 als PC gekennzeichnet. Zum Erreichen von optischer Einheitlichkeit der Schicht FLC wurde ein Polyvinyl-Alkohol-Film auf kreisrunde Substrate mit einem Durchmesser von 35 mm mittels eines Spincoating-Verfahrens aufgebracht. Nachdem der polymere Film bei 130°C behandelt wurde, wurde er mit einem Baumwolltuch in einer Richtung gerieben. Der Zellenabstand $FLC_1$ und $FLC_2$ betrug ca. 5.5μm, wodurch sich die optische Bedingung $\Delta n \cdot d = 3\,\lambda/2$ für den grünen Bereich des Lichts ergibt.

Die $FLC_1$ und $FLC_2$ Zellen wurden in der isotropen Phase des FLC-Materials mit diesem befüllt und auf Raumtemperatur abgekühlt. Die Basisparameter des verwendeten DHF-Materials sind:

> Phasenübergänge Kr. ( < -10°C) $S^*_c$ (59°C) Is,
> spontane Polarisation $P_s \approx 200$ nC.cm$^{-2}$,
> Helixsteigung $p_o \approx 0.27$μm,
> Neigungswinkel $\theta_o = 31°$ (20°C).

Bei beiden FLC-Schichten wurde hohe optische Einheitlichkeit (der durchschnittliche Kontrast betrug 200 auf einer Fläche von > 5cm$^2$) bei gleichzeitiger Anwendung von Wechselspannung mit einer Amplitude von ± 50 V und einer Frequenz von 10-1000 Hz bei exaktem Scheeren der Substrate gegeneinander, senkrecht zur Reibrichtung, erzielt. In diesem Fall hat die FLC-Schicht genau die Geometrie eines Regals (bookshelf) für relativ kleine Werte spontaner Polarisation oder die einer "dislocation domain structure" bei höherer spontaner Polarisation gemäß der Beschreibung von L. A. Beresnev, E. Schumacher, S.A. Pikin, Z. Fan, B.I. Ostrovsky, S. Hiller, A.P. Onokhov und W. Haase in Jpn. J. Appl. Phys., Band 34, Teil 1, Nr. 5A, vom Mai 1995.

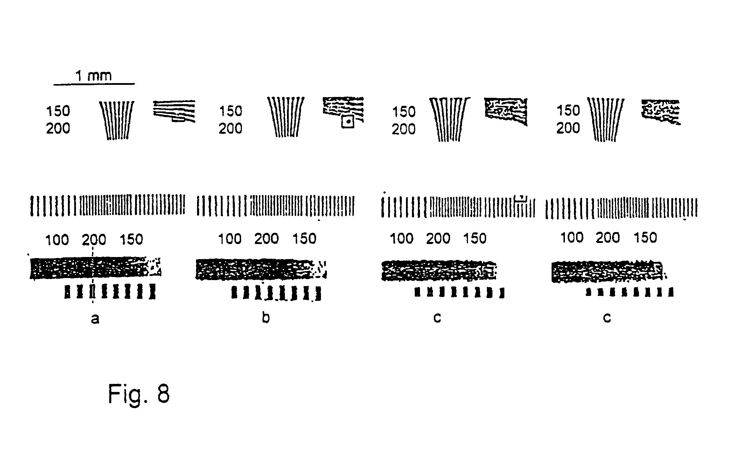

In Fig. 8a ist ein ausgewähltes Bild des Testzielbildes 0683 dargestellt, fokussiert auf den optisch adressierbaren räumlichen Lichtmodulator OASLM, basierend auf ZnSe+DHF; die Dicke der FLC-Schicht beträgt 5.5μ, die Amplitude der angelegten Wechselspannung (Mäander) ist ± 7.5 V, deren Frequenz 50 Hz. Die Größe des Bildes beträgt 1.5 x 1.5 mm. Die Nummern in den Bildern 100, 200, 150 entsprechen der räumlichen Auflösung 20, 40 bzw. 30 pl/mm. (Fig. 8b-d) - ausgewähltes Bild des Testzielbildes, registrierbar mit sich bewegendem optisch adressierbaren räumlichen Lichtmodulator OASLM. Der optisch adressierbare räumliche Lichtmodulator OASLM bewegt sich von oben nach unten. Fig. 8b - 1.5 mm/Sek., Fig. 8c - 4.5 mm/Sek., Fig. 8d - 6.5 mm/Sek.. Das Intervall zwischen den Bildern beträgt 40 ms. Der Punkt im Quadrat gehört zum optisch adressierbaren räumlichen Lichtmodulator OASLM und dient als Bezugspunkt.

In Fig. 8 werden die grundsätzlichen Merkmale des hergestellten optisch adressierbaren räumlichen Lichtmodulators OASLM beschrieben. Das blaue Licht wurde zum Aufzeichnen des Testzielbildes und das rote Licht zum Auslesen des sich ergebenden Bildes benutzt. Es wurde eine Rechteck-Betriebsspannung (Mäander) mit einer Amplitude von ± 7.5 V und einer Frequenz von 50 Hz angewendet. Die räumliche Auflösung des so erzielten optisch adressierbaren räumlichen Lichtmodulators OASLM ist höher als 40 pl/mm bei einer Modulationstiefe von ca. 50%. Die Auflösung für nur eine Polarität der Betriebsspannung kann höher als 100 pl/mm bei derselben Modulationstiefe sein. Der Grenzwert wird durch das optische Schema der Projektion begrenzt.

In den Fig. 8b-d werden die "read-out" Bilder bei Bewegung des optisch adressierbaren räumlichen Lichtmodulators OASLM bezüglich des Testzielbildes dargestellt. Hier zeigt sich, daß bis zu einer Bewegungsgeschwindigkeit von 8 mm/s das Bild relativ wenig verwackelt (verwischt) ist, was auch auf das Videoaufzeichnungsverfahren zurückgeführt werden kann.

Zum Testen der Funktionsweise der Brille wird eine Wolfram-Halogenlampe von 100 W als sehr helles Bild eingesetzt. Hinter die Lampe wird ein weißes Papier mit einer Aufschrift ("goggles") gebracht. Zwischen Linse $L_3$ und Videokamera wird ein grünes Filter geschaltet, um die infraroten und ultravioletten Bereiche des Lichtspektrums zu unterdrücken, die durch die Polarisatoren $P_1$ und $P_3$ treten. Die optischen Parameter bei Verschluß waren: $f_1$ = 5 cm, $f_2$ = 2 cm und $f_3$ = 1cm (Fig. 1).

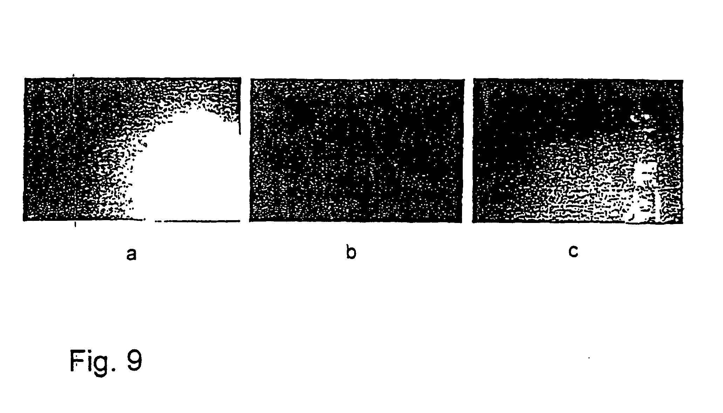

Fig. 9 zeigt die Arbeitsweise der Sicherheitsvorrichtung mit lokaler Abschwächung der Helligkeit von Bildern stark belichteter Objekte unter Verwendung von optisch adressierbaren räumlichen Lichtmodulatoren OASLM auf der Grundlage eines photoleitenden Films (ZnSe), Dicke ∼1μm, unter Ausnutzung des DHF-Effektes in FLC's, Dicke der FLC Schicht 5.5 μm. Schema "mit Verschluß"; (siehe Fig. 3 und 4; a - keine Spannung auf dem optisch adressierbaren räumlichen Lichtmodulator OASLM, Verschluß ist geöffnet, starkes Blenden der Videokamera; b - Verschluß ist geschlossen, der Leuchtfaden ist nur sehr schwach zu sehen; c - der Verschluß ist geöffnet, die Spannung am optisch adressierbaren räumlichen Lichtmodulator OASLM beträgt +10 V, die Helligkeit des Leuchtdrahtes wird stark unterdrückt, jedoch ist das Objekt hinter der Lampe (Papier mit dem Wort "Brille") zu sehen, ohne daß dabei eine Blendung auftritt; die Mäanderspannung beträgt ± 10 V, 1 Hz).

In Fig. 9 wird gezeigt, daß während der Anwendung von elektrischer Spannung auf den optisch adressierbaren räumlichen Lichtmodulator OASLM die Helligkeit von sehr stark beleuchteten Objekten (Glühdraht) tatsächlich unterdrückt wird, während die Helligkeit von schwächer beleuchteten Objekten (Papier mit dem Wort "goggles") nicht unterdrückt wird. Der Text ist also gut sichtbar, ohne daß die Videokamera geblendet wird. Zum Vergleich wird das gleiche Bild gegenübergestellt, wenn an den optisch adressierbaren räumlichen Lichtmodulator OASLM keine Spannung angelegt wird. In diesem Fall ist die Helligkeit der Lampe zu stark, um den Text zu erkennen zu können, da die Videokamera geblendet wird.

Damit die Vorrichtung reibungslos arbeitet, schließt die Blende $FLC_2$ der Vorrichtung 5 ÷ 10 ms nach Reaktion des optisch adressierbaren räumlichen Lichtmodulators OASLM. Gleichzeitig ändert sich die Polarität der an den optisch adressierbaren räumlichen Lichtmodulator OASLM angelegten Spannung. Der gesamte Zyklus wird nach 10 ÷ 20 ms wiederholt. Die Vorrichtung arbeitet bis zu einer Mäanderfrequenz von 1 kHz, wenn die Feineinstellung für das Vorspannungsfeld erfolgt ist und der optisch adressierbare räumliche Lichtmodulator OASLM sich zwischen den gekreuzten Polarisatoren $P_1$ und $P_2$ befindet. Diese hohen Frequenzen bieten die Möglichkeit, die Bewegung von schwach beleuchteten Objekten vor dem Hintergrund von stark beleuchteten Objekten, wie Sonne, Lampen u.s.w. zu beobachten, ohne daß die Bilder verschwimmen und Auge oder Videokamera 1 geblendet werden. Die Vorrichtung kann nicht nur erfolgreich zur Realisierung von optischen Reglern in optischen Telekommunikationsleitungen eingesetzt werden, sondern auch bei Schweiß- und Schneidbrennarbeiten, sowie bei anderen Tätigkeiten, bei denen hohe lokale Lichtintensitäten auftreten.

**Patentansprüche**

1. Vorrichtung zur lokalen Abschwächung der Lichtintensität im Sehfeld des menschlichen Auges, von Video- oder Photokameras oder dergleichen bzw. von lichtempfindlichen Vorrichtungen zum Beobachten und Aufzeichnen von beleuchteten Bildern, bestehend aus Linsen und Filtern, wobei zwischen einem Objektiv aus einer oder mehreren Linsen ($L_1$) und zwei gekrenzten Polarisatoren ($P_1$ und $P_2$) ein optisch adressierbarer räumlicher Lichtmodulator (OASLM) angeordnet ist, der zwei transparente Platten mit Elektroden und eine halbtransparente photoleitende Schicht (PC) umfasst, **dadurch gekennzeichnet, dass** der Lichtmodulator (OASLM) mit einem helixförmigen, ferroelektrischen, smektischen Flüssigkristall (FLC) versehen ist, der zwischen den Platten in Buchregal-Geometrie und/oder Deformationsdomänen-Geometrie angeordnet ist, die Helixachse des smektischen Flüssigkristalls (FLC) parallel zu den Platten verläuft und der molekulare Neigungswinkel $\theta_o$ der Flüssigkristallschicht (FLC) 45 ° beträgt, und die Reibrichtung z mit dem Polarisator ($P_1$) einen Winkel von 45° bildet.

2. Vorrichtung zur lokalen Abschwächung der Lichtintensität im Sehfeld des menschlichen Auges, von Video- oder Photokameras oder dergleichen bzw. von lichtempfindlichen Vorrichtungen zum Beobachten und Aufzeichnen von beleuchteten Bildern, bestehend aus Linsen und Filtern, wobei zwischen einem Objektiv aus einer oder mehreren Linsen ($L_1$) und zwei gekrenzten Polarisatoren ($P_1$ und $P_2$) ein optisch adressierbarer räumlicher Lichtmodulator (OASLM) angeordnet ist, der zwei transparente Platten mit Elektroden und eine halbtransparente photoleitende Schicht (PC) umfasst, **dadurch gekennzeichnet, dass** der Lichtmodulator (OASLM) mit einem helixförmigen, ferroelektrischen, smektischen Flüssigkristall (FLC) versehen ist, der zwischen den Platten in Buchregal-Geometrie und/oder Deformationsdomänen-Geometrie angeordnet ist, die Helixachse des smektischen Flüssigkristalls (FLC) parallel zu den Platten verläuft, und dass zum Verhindern der Übertragung von Licht in der Vorrichtung für die Gegenpolarität der auf die genannten Elektroden angelegten Spannung ein Verschluss (2) angeordnet ist.

3. Vorrichtung zur lokalen Abschwächung der Lichtintensität im Sehfeld des menschlichen Auges, von Video- oder Photokameras oder dergleichen bzw. von lichtempfindlichen Vorrichtungen zum Beobachten und Aufzeichnen von beleuchteten Bildern, bestehend aus Linsen und Filtern, wobei zwischen einem Objektiv aus einer oder mehreren Linsen ($L_1$) und zwei gekrenzten Polarisatoren ($P_1$ und $P_2$) ein optisch adressierbarer räumlicher Lichtmodulator (OASLM) angeordnet ist, der zwei transparente Platten mit Elektroden und eine halbtransparente photoleitende Schicht (PC) umfasst, **dadurch gekennzeichnet, dass** der Lichtmodulator (OASLM) mit einem helixförmigen, ferroelektrischen, smektischen Flüssigkristall ($FLC_1$) versehen ist, der zwischen den Platten in Buchregal-Geometrie und/oder Deformationsdomänen-Geometrie angeordnet ist, die Helixachse des smektischen Flüssigkristalls ($FLC_1$) parallel zu den Platten verläuft, und dass eine zweite ferroelektrische chirale smektische Flüssigkristallschicht ($FLC_2$), die sich zwischen zwei weiteren transparenten mit Elektroden versehenen Platten befindet, zwischen dem optisch adressierbaren räumlichen Lichtmodulator (OASLM) und einem der genannten Polarisatoren ($P_1$, $P_2$) angeordnet ist.

4. Vorrichtung nach Patentanspruch 3, **dadurch gekennzeichnet, dass** in einem stark belichteten Zustand bei beiden an die Elektroden angelegten Spannungspolaritäten die optischen Eigenschaften des zweiten ferroelektrischen, chiralen, smektischen Flüssigkristalls ($FLC_2$) zugesteuert sind, sodass die Lichtübertragung in der Vorrichtung gesperrt ist.

5. Vorrichtung nach einem der Patentansprüche 3 oder 4, **dadurch gekennzeichnet, dass** sich der zweite ferroelektrische, smektische Flüssigkristall ($FLC_2$) in der ferroelektrischen smektischen C-Phase mit einem molekularen Neigungswinkel von 45°-$\theta_o$ oder $\theta_o$, befindet, wobei $\theta_o$ der molekulare Neigungswinkel des genannten ferroelektrischen smektischen Flüssigkristalls ($FLC_1$) im optisch adressierbaren räumlichen Lichtmodulator (OASLM) ist.

6. Vorrichtung nach Anspruch 4, **dadurch gekennzeichnet, dass** der zweite smektische Flüssigkristall ($FLC_2$) sich in der chiralen smektischen A-Phase befindet.

7. Vorrichtung nach einem der vorangehenden Patentansprüche, **dadurch gekennzeichnet, dass** sich der ferroelektrische smektische Flüssigkristall (FLC, $FLC_1$) in der geneigten smektischen C-Phase befindet.

8. Vorrichtung nach Patentanspruch 2, **dadurch gekennzeichnet, dass** der Verschluss (2) als weitere ferroelektrische Flüssigkristallschicht ($FLC_2$) ausgebildet ist, die sich ihrerseits zwischen zwei transparenten, mit Elektroden versehenen Platten befindet und zwischen einer der genannten polarisierenden Schichten bzw. Polarisatoren ($P_1$, $P_2$) und einer dritten polarisierenden Schicht bzw. einem Polarisator ($P_3$) positioniert ist.

9. Vorrichtung nach Patentanspruch 9, **dadurch gekennzeichnet, dass** der zweite ferroelektrische Flüssigkristall ($FLC_2$) sich in einer chiralen smektischen C- oder smektischen A-Phase befindet.

**Claims**

1. Device for local attenuation of the light intensity in the visual field of the human eye, of video or photo cameras or similar or of light-sensitive devices for the viewing and recording of illuminated images, consisting of lenses and filters, wherein disposed between an objective comprising one or more lenses ($L_1$) and two crossed polarizers ($P_1$ and $P_2$) is an optically addressable spatial light modulator (OASLM) comprising two transparent plates with electrodes and a semi-transparent photoconductive layer (PC), **characterized in that** the light modulator (OASLM) is provided with a helical, ferroelectric, smectic liquid crystal (FLC) disposed between the plates in bookshelf geometry and/or deformation domain geometry, the helical axis of the smectic liquid crystal (FLC) extending parallel to the plates and the molecular inclination angle $\theta_o$ of the liquid crystal layer (FLC) being 45°, and the friction direction z forming an angle of 45° with the polarizer ($P_1$).

2. Device for local attenuation of the light intensity in the visual field of the human eye, of video or photo cameras or similar or of light-sensitive devices for the viewing and recording of illuminated images, consisting of lenses and filters, wherein disposed between an objective comprising one or more lenses ($L_1$) and two crossed polarizers ($P_1$ and $P_2$) is an optically addressable spatial light modulator (OASLM) comprising two transparent plates with electrodes and a semi-transparent photoconductive layer (PC), **characterized in that** the light modulator (OASLM) is provided with a helical, ferroelectric, smectic liquid crystal (FLC) disposed between the plates in bookshelf geometry and/or deformation domain geometry, the helical axis of the smectic liquid crystal (FLC) extending parallel to the plates, and **in that**, in order to prevent the transmission of light, a shutter (2) is disposed in the device for the opposing polarity of the voltage applied to the aforementioned electrodes.

3. Device for local attenuation of the light intensity in the visual field of the human eye, of video or photo cameras or similar or of light-sensitive devices for the viewing and recording of illuminated images, consisting of lenses and filters, wherein disposed between an objective comprising one or more lenses ($L_1$) and two crossed polarizers ($P_1$ and $P_2$) is an optically addressable spatial light modulator (OASLM) comprising two transparent plates with electrodes and a semi-transparent photoconductive layer (PC), **characterized in that** the light modulator (OASLM) is provided with a helical, ferroelectric, smectic liquid crystal (FLC) disposed between the plates in bookshelf geometry and/or deformation domain geometry, the helical axis of the smectic liquid crystal (FLC) extending parallel to the plates, and **in that** a second ferroelectric chiral smectic liquid crystal layer ($FLC_2$) situated between two further transparent plates provided with electrodes is disposed between the optically addressable spatial light modulator (OASLM) and one of the aforementioned polarizers ($P_1$, $P_2$).

4. Device according to claim 3, **characterized in that**, in a state heavily exposed to light, with both voltage polarities applied to the electrodes, the optical properties of the second ferroelectric, chiral, smectic liquid crystal ($FLC_2$) are closed, with the result that the transmission of light in the device is blocked.

5. Device according to any one of claims 3 or 4, **characterized in that** the second ferroelectric, smectic liquid crystal ($FLC_2$) is in the ferroelectric, smectic C-phase with a molecular inclination angle of 45°-$\theta_o$ or $\theta_o$, where $\theta_o$ is the molecular inclination angle of the aforementioned ferroelectric, smectic liquid crystal ($FLC_1$) in the optically addressable spatial light modulator (OASLM).

6. Device according to claim 4, **characterized in that** the second smectic liquid crystal ($FLC_2$) is in the chiral smectic A-phase.

7. Device according to any one of the preceding claims, **characterized in that** the ferroelectric smectic liquid crystal ($FLC_1$) is in the inclined smectic C-phase.

8. Device according to claim 2, **characterized in that** the shutter (2) is in the form of a further ferroelectric liquid crystal layer ($FLC_2$) which, in turn, is situated between two transparent plates provided with electrodes and is positioned between one of the aforementioned polarizing layers or polarizers ($P_1$, $P_2$) and a third polarizing layer or polarizer ($P_3$).

9. Device according to claim 9, **characterized in that** the second ferroelectric liquid crystal ($FLC_2$) is in a chiral smectic C- or smectic A-phase.

**Revendications**

1. Dispositif d'atténuation locale de l'intensité lumineuse, dans le champ de vision de l'oeil humain, pour des caméras vidéo, des appareils photographiques ou similaires ou des appareils photosensibles d'observation et d'enregistrement de figures éclairées, constitué de lentilles et de filtres, dans lequel, entre un objectif constitué d'une ou plusieurs lentilles ($L_1$) et deux polariseurs à prismes croisés ($P_1$ et $P_2$), est disposé un modulateur de lumière spatial optiquement adressable (OASLM), qui comprend deux plaques transparentes dotées d'électrodes et une couche photoconductrice semi-transparente (PC),
**caractérisé en ce que**
le modulateur de lumière (OASLM) est pourvu d'un cristal liquide (CL) smectique, ferroélectrique, en forme d'hélice, qui est disposé entre les plaques suivant une géométrie d'empilement et/ou une géométrie des domaines de déformation, l'axe de l'hélice du cristal liquide smectique (CL) est parallèle aux plaques, l'angle d'inclinaison moléculaire $\theta_0$ de la couche de cristal liquide (CL) est de 45° et le sens de frottement z forme avec le polariseur ($P_1$) un angle de 45°.

2. Dispositif d'atténuation locale de l'intensité lumineuse dans le champ de vision de l'oeil humain, pour des caméras vidéo, des appareils photographiques ou similaires ou des appareils photosensibles d'observation et d'enregistrement de figures éclairées, constitué de lentilles et de filtres, dans lequel, entre un objectif constitué d'une ou plusieurs lentilles ($L_1$) et deux polariseurs à prismes croisés ($P_1$ et $P_2$), est disposé un modulateur de lumière spatial optiquement adressable (OASLM), qui comprend deux plaques transparentes dotées d'électrodes et une couche photoconductrice semi-transparente (PC),
**caractérisé en ce que**
le modulateur de lumière (OASLM) est pourvu d'un cristal liquide (CL) smectique, ferroélectrique, en forme d'hélice, qui est disposé entre les plaques suivant une géométrie d'empilement et/ou une géométrie des domaines de déformation, l'axe de l'hélice du cristal liquide smectique (CL) est parallèle aux plaques et, pour empêcher la propagation de la lumière, un élément d'obturation (2) est prévu dans le dispositif pour assurer la contre-polarité de la tension appliquée aux électrodes mentionnées.

3. Dispositif d'atténuation locale de l'intensité lumineuse dans le champ de vision de l'oeil humain, pour des caméras vidéo, des appareils photographiques ou similaires ou des appareils photosensibles d'observation et d'enregistrement de figures éclairées, constitué de lentilles et de filtres, dans lequel, entre un objectif constitué d'une ou plusieurs lentilles ($L_1$) et deux polariseurs à prismes croisés ($P_1$ et $P_2$), est disposé un modulateur de lumière spatial optiquement adressable (OASLM), qui comprend deux plaques transparentes dotées d'électrodes et une couche photoconductrice semi-transparente (PC),
**caractérisé en ce que**
le modulateur de lumière (OASLM) est pourvu d'un cristal liquide (CL) smectique, ferroélectrique, en forme d'hélice, qui est disposé entre les plaques suivant une géométrie d'empilement et/ou une géométrie des domaines de déformation, l'axe de l'hélice du cristal liquide smectique ($CL_1$) est parallèle aux plaques et une deuxième couche de cristal liquide smectique, chiral, ferroélectrique ($CL_2$), qui se trouve entre deux autres plaques transparentes dotées d'électrodes, est disposée entre le modulateur de lumière spatial optiquement adressable (OASLM) et un des polariseurs mentionnés ($P_1$, $P_2$).

4. Dispositif selon la revendication 3, **caractérisé en ce que**, dans une condition de fort éclairage, pour les deux polarités de tension appliquées aux électrodes, les propriétés optiques du second cristal liquide ($CL_2$) smectique, chiral, ferroélectrique sont réglées de façon à ce que toute transmission de lumière dans le dispositif soit empêchée.

5. Dispositif selon l'une des revendications 3 ou 4, **caractérisé en ce que** le second cristal liquide ($CL_2$) smectique, ferroélectrique se trouve dans la phase C smectique ferroélectrique avec un angle d'inclinaison moléculaire de 45° - $\theta_0$ ou $\theta_0$, $\theta_0$ représentant l'angle d'inclinaison moléculaire dudit cristal liquide ($CL_1$) smectique ferroélectrique dans le modulateur de lumière spatial optiquement adressable (OASLM).

6. Dispositif selon la revendication 4, **caractérisé en ce que** le second cristal liquide smectique ($CL_2$) se trouve dans la phase smectique A chirale.

7. Dispositif selon l'une des revendications précédentes, **caractérisé en ce que** le cristal liquide smectique ferroélectrique ($CL_1$) se trouve dans la phase smectique C inclinée.

8. Dispositif selon la revendication 2, **caractérisé en ce que** l'élément d'obturation (2) est conçu sous forme d'une autre couche de cristal liquide ferroélectrique ($CL_2$) qui se trouve pour sa part entre deux plaques transparentes dotées d'électrodes et qui est positionnée entre une des couches polarisantes ou polariseurs mentionnés ($P_1$ et $P_2$) et une troisième couche polarisante ou un polariseur ($P_3$).

**9.** Dispositif selon la revendication 9, **caractérisé en ce que** le second cristal liquide ferroélectrique ($CL_2$) se trouve dans une phase smectique C chirale ou dans une phase smectique A.

Fig. 1

Fig. 2

Fig. 3

Fig. 4

Fig. 5

Fig. 6

Fig. 7

Fig. 8

a                          b                          c

Fig. 9